Europäisches Patentamt

European Patent Office

Office européen des brevets

(19)

(11) Veröffentlichungsnummer: **0 043 075**

**A2**

(12)

# EUROPÄISCHE PATENTANMELDUNG

(21) Anmeldenummer: **81104827.1**

(22) Anmeldetag: **23.06.81**

(51) Int. Cl.³: **C 12 N 15/00**
**C 12 P 21/00, C 12 N 9/86**
**C 12 N 9/36, C 12 N 9/12**

(30) Priorität: **25.06.80 DE 3023787**

(43) Veröffentlichungstag der Anmeldung:
**06.01.82 Patentblatt 82/1**

(84) Benannte Vertragsstaaten:
**AT BE CH DE FR GB IT LI LU NL SE**

(71) Anmelder: **Studiengesellschaft Kohle mbH**
**Kaiser-Wilhelm-Platz 1**
**D-4330 Mülheim/Ruhr(DE)**

(72) Erfinder: **Nicolau, Yves-Claude, Prof. Dr.**
**Leonhard-Stinnes-Strasse 48**
**D-4330 Mülheim/Ruhr(DE)**

(72) Erfinder: **Senè, Claude, Dr.**
**Jahnstrasse 3**
**Mülheim/Ruhr(DE)**

(74) Vertreter: **von Kreisler, Alek, Dipl.-Chem. et al,**
**Deichmannhaus am Hauptbahnhof**
**D-5000 Köln 1(DE)**

(54) Verfahren zur Erhöhung der Inkorporation und der Expression von genetischem Material in die Kerne von intakten Zellen mit Hilfe von Liposomen.

(57) Ein Verfahren zur Erhöhung der Aufnahme und der Expression von genetischem Material in die Kerne von intakten Zellen ist dadurch gekennzeichnet, daß man mit genetischem Material beladene Endozytose-Liposome durch Endozytose in die Zellkerne einführt.

EP 0 043 075 A2

Croydon Printing Company Ltd.

# Verfahren zur Erhöhung der Inkorporation und der Expression von genetischem Material in die Kerne von intakten Zellen mit Hilfe von Liposomen

Liposomen wurden benutzt, um genetisches Material in Zellen einzubauen (Poste, G., und Papahadjopoulos, D; Proc. Natl. Acad. Sci. USA (1976) 73, 1603-1607; Weissmann, G. Bloemgarden, D., Kaplan, R., Hoffstein, S., Collins, T., Gotlieb, A. und Nagle, D.; Proc. Natl. Acad. Sci. USA (1975) 72, 88 - 92; Poste, G., Papahadjopoulos, D., und Vail, W.J., (1976) Methods in Cell Biology ed. Prescott, D. Academ. Press, New York, vol. XIV, 33 - 71; Gregoradis, G., (1974) in Enzyme Terapy in Lysosomal Storage Diseases, eds. Tager J.M., Hooghwinkel G.J.M., und Daems, W.Th. (North Holland) Amsterdam pp. 131 - 148). In der Patentanmeldung P 29 42 780.3 wurde die Inkorporation des bla-Gens in verschiedenen eukaryoten Zellen und dessen Expression vorgeschlagen. Dort wurde die Inkorporation eines Restriktionsfragments aus dem Plasmid pBR 322 in verschiedene Zellen (Hela, LM, Human-Lymphocyten, Human-Hepatozyten, Hühner,Embryofibroblasten) mit Hilfe von DNS-beladenen Liposomen beschrieben. Die erzielte DNS-Inkorporation in den aufgezählten Zellen erreichte 2,5 % des Ausgangsmaterials, d.h. maximal 2000 Genkopien pro Zelle. Die Genaufnahme im Kern der Wirtszellen erreichte maximal 37 % dieser Zahl.

Die vorliegende Erfindung stellt sich die Aufgabe, die Genaufnahme in den Zellkernen und das Niveau der Expression signifikant zu erhöhen. Darüber hinaus soll ein Gen-Dosierungseffekt bewirkt werden, der auch mit Hilfe der Liposomen

- 2 -

zustande gebracht werden kann. Die Erfindung betrifft die in den Ansprüchen dargelegten Gegenstände. Das erfindungsgemäße Verfahren wurde mit folgenden Genen durchgeführt:

1.) Restriktionsfragmente des bla-Gens aus dem Plasmid pBR 322 E-coli
(Sutcliffe, I.G.; Proc. Nat. Acad. Sci. USA (1978) 75 3737 - 3741)

2.) Das gesamte pBR 322 E-coli Plasmid
(Fraley R.T.; Fornari, C.S. und Kaplan, S. Proc. Nat. Acad. Sci. USA (1979) 76 3348 - 3352

3.) Das Kaninchen ß-Globin Gen in pBR 322 Plasmid
(Maniatis, T.; Kee, S.G., Efstratiadis, A. und Kafatos, F.C. (1976) Cell 8 163 - 182)

4.) Die Lysozym c-DNA im gesamten pBR Plasmid
(Sippel, A.E.; Land, H.; Lindenmaier, W.;Minhchi Nguyen-HUU, MC.; Wurtz, T.; Timmth, K.N.; Giesecke, K.; Schütz, G.;
Nucleic Acid Research (1978) 5 3275 - 3298)

5.) Das Conalbumin-Gen im pBR 322 Plasmid
(Wasylyk, B.; Kedinger, C.; Corden, I.; Brison, O &; Chambon, P.; (1980) Nature 285 367 - 373)

6.) Das Thymidinkinase-Gen im pBR 322 Plasmid
(Colbere-Garapin, F.; Choustermon, S.; Horodniceau, F.; Kourilsky, P.; Garapin, A.C.; Proc. Nat. Acad. Sci. USA (1979) 76 3755 - 3759)

7.) Das SV 40 Virus
(Goff, S.P.; Berg, P.; Cell. 9, 695 - 705 (1976) )

8.) Das genomiale Interferon-Gen in pBR 322 Plasmid
(Nagata, S.; Taira, H.; Hall, A.; Johnsrud, L.;
Streuli, M.; Escodi, J.; Boll, W.; Cantell, K.;
Weissmann, C.; Nature (1980) 284, 316 - 320)

9.) Die c-DNA des Interferons im Plasmid
(vergl. 8.))

10.) Das Insulin-Gen im Plasmid pBR 322
(Seeburg, P.H.; Shine, J.; Martial, I.A.; Ivarie, R.D.;
Morris, I.A.; Ullrich, A.; Baxter, J.D.; Goodman, H.M.;
(1978) Nature 276 795 - 798)

Es ist bekannt, daß fluide, elektrisch geladene Liposomen hauptsächlich durch Fusion in Kulturzellen dringen, während fluide, neutrale oder feste, elektrisch geladene Liposomen durch Endozytose in Kulturzellen eindringen. Während der Fusion gleichzeitig mit der Verschmelzung der äußeren Lipidschichten von zwei Membranen findet auch die Entladung des eingeschlossenen Materials statt. Bei der Endozytose von Liposomen durch Zellen werden die Vesikel als intakte Strukturen von den Zellen aufgenommen und im Inneren der Zellen erst durch Wechselwirkungen mit den lysozomalen Enzymen geöffent. Das Zell-Zytoplasma ist reich an DNasen. Die DNS-Moleküle, die durch die Fusion von DNS-beladenen Liposomen mit den Zellen in das Zytoplasma gelangen, werden in großem Ausmaß der Einwirkung dieser DNasen ausgesetzt. Sollten aber die DNS-Moleküle nach ihrem Eindringen in die Zellen mindestens eine Zeitlang geschützt werden, würde sich die Wahrscheinlichkeit erhöhen, daß eine größere Zahl solcher Moleküle den Zellkern erreichen.

Dieses kann erfindungsgemäß durch die Verwendung von Endozytose Liposomen erreicht werden. Weiterhin wurde jetzt

- 4 -

gefunden, daß die Zahl der DNS-Moleküle, die in den Zellkern aufgenommen werden kann, auch vom Zustand des Kerns abhängt. In der mitotischen Phase des Zellzyklus ist der Kern von keiner Membran umgeben; gleichzeitig finden in der mitotischen Phase die meisten "Genrearrangements" statt.

Erfindungsgemäß wurden die Zellen in den verschiedenen Zellzyklusphasen synchronisiert und die Aufnahme des fremden genetischen Materials in die Zellkerne bestimmt. Weiterhin wurden erfindungsgemäß verschiedene Konzentrationen von Genmaterial in die Zellen eingebaut und die dabei ablaufende Proteinproduktion analytisch bestimmt. Dies erlaubte die Beobachtung, daß im Konzentrationsbereich 200 - 1200 Genmoleküle/Zelle ein starker Anstieg der Proteinproduktion stattfindet; zwischen 1200 und 2000 Genmolekülen/Zelle wird diese Produktion für die untersuchten Zellen weitgehend konstant.

Beispiel 1

a) Zellen: Die folgenden Zellen wurden verwendet: Hela, Human DG 75 Lymphozyten, Maus LMTK⁻, Hühner-Embryofibroblasten, Human-Knochenmarkzellen, Detroit 6 BHK 21 Hamsterzellen, Human-Hepatozyten und Human WI-38 Fibroblasten. Die Zellen wurden in üblicher Weise, z.B. wie in der oben zitierten Literatur beschrieben, kultiviert.

b) Genetisches Material

1. bla-Gen
2. Plasmid pBR 322 von E. coli
3. Plasmid pBR 322 von E.coli mit dem Kaninchen-ß-globin-Gen
4. Das Gen des Hühner-Conalbumin im Plasmid
5. Das C-DNA des Hühner-Lysozyms auch im Plasmid.

Inkorporation der Gene in Liposome

Für das Einschließen großer DNS-Moleküle als ganze Plasmide in Liposome wurde eine Injektionsmethode (Lurquin, P.F., (1979) Nucleic Acid. Res. 6, 3773-3784) benutzt. Mit dieser Methode wurde eine Ätherlösung der gewünschen Lipide gleichmäßig in eine auf 60°C erwärmte DNS-Lösung eingespritzt. Es bilden sich große Vesikel, die die DNS-Moleküle einschließen. Zwei Klassen solcher Vesikel wurden hergestellt:

1. Endozytose-Liposome, bestehend aus Phosphatidylcholin aus Eigelb (PC)
2. Fusion-Liposome, bestehend aus PC und geladenen Lipiden, wie: Phosphatidyl-serin, Phosphatidsäure, Stearylamin.

Die Lipidzusammensetzung der Liposome hängt immer auch von der Lipidzusammensetzung der Zellmembrane ab, mit der die Wechselwirkung stattfinden sollte.

Die erhaltenen Liposome, mit DNS beladen, wurden anschließend 30 Minuten mit 50 $\mu$g/ml DNase I bei 37°C inkubiert, um die nicht eingeschlossenen Moleküle zu hydrolisieren. Eine Gelfiltration auf Sepharose/4B zeigte dann, daß allein die eingeschlossene DNS intakt geblieben war.

Die benutzten Lipidkonzentrationen, um DNS-beladene Liposome zu erhalten, variierten von 1,4 $\mu$g/ml bis zu 7 mg/ml je nach Zahl der Zellen, die inkubiert werden sollten und nach dem Konzentrationsverhältnis DNS : Lipide. Das Ergebnis zeigt überraschend, daß sehr große DNS-Moleküle (bis zum Molekulargewicht von $18 \times 10^6$ Dalton, aber auch darüber hinaus) als intaktes genetisches Material in Liposome eingeschlossen werden können.

- 6 -

## Zell-Liposom Wechselwirkung

Die DNA-beladenen Liposome werden mit Hela-Zellen, Hühnerembryozellen, WI-38 Human-Fibroblasten, Hepatozyten (human) oder Lymphozyten inkubiert. Ehe 4 ml der Liposom-Suspension zu $10^7$ Zellen zugegeben werden, werden die Zellen mit 20ml DMEM ohne Serum gewaschen. Nach einer Inkubation von 2 Stunden bei 37°C wurde die Liposom-Suspension dekantiert und die Zellen zweimal mit 10 ml PBS gewaschen, bevor das serumfreie DMEM zugesetzt wurde. Um nicht eingeschlossene DNA, die an der Zelloberfläche adsorbiert ist, zu entfernen, werden die Zellen mit DNase I behandelt. Nach Entfernen der Liposom-Suspension durch Wäsche werden aliquote Teile der Zellen mit 4 ml einer DNase I-Lösung (50 $\mu$g/ml in PBS mit 10 mM $MgCl_2$) versetzt, vermischt und die Mischung während 30 Minuten inkubiert.

## Extraktion der Zell-Nuklearsäuren

Nach Inkubation wurden die Zellen mit Phosphatbufferdsaline (PBS) gewaschen, trypsiniert und pelletiert durch Zentrifugation. Die Zellen wurden im RBS-Puffer suspendiert (1o mMol NaCl, 10 mMol Tris.HCl pH = 7,5, 1,5 mMol $MgCl_2$) und 1 % Triton x-100 hinzugefügt. Die Zellen wurden durch Homogenisation in einem Dounce-Homogenisator lysiert. Die Zellkerne wurden durch 5 Minuten Zentrifugation bei 1000 Ud/N pelletiert und zweimal mit dem RBS-Puffer gewaschen. Die Lyse der Zellen wurde in Anwesenheit von 300 $\mu$g/ml kalter DNS durchgeführt.

Durch die Behandlung der Zellen mit 2 % Na-dodecylsulfat in 0,1 M Tris.HCl, pH = 9,0, 0,2 n NaCl, und 1-stündiger Inkubation mit 100 mg/ml Proteinase K bei 37°C wurden die totalen DNS-Extrakte erhalten. Die Entfernung der Proteine erfolgte durch zweimaliges Mischen mit Phenol. Die DNS wurde mit $CCl_3CO_2H$ prezipitiert, auf GF/C Glasfaserfilter aufgetragen und mit 5 % Trichloressigsäure (TCA) und 95 % Äthanol gewaschen.

Synchronisierung der Zellen

Die Zellen wurden, wie von Senè, C. et al (FEBS Lett. 88, 187 - 191, 1978) beschrieben, durch Thymidinblock synchronisiert.

Bestimmung des Genproduktes

a) ß-Lactamase (bla) wie beschrieben von O' Callaghan, C.H. Morris, A. Kirby, S.M., and Shingler, A.H. (1972) Antimicrobial Agents und Chemotherapy 1, 283 - 288.

b) ß-Globin, beschrieben von Ostro et al (Nature 274, 921-923, 1978).

Ergebnisse

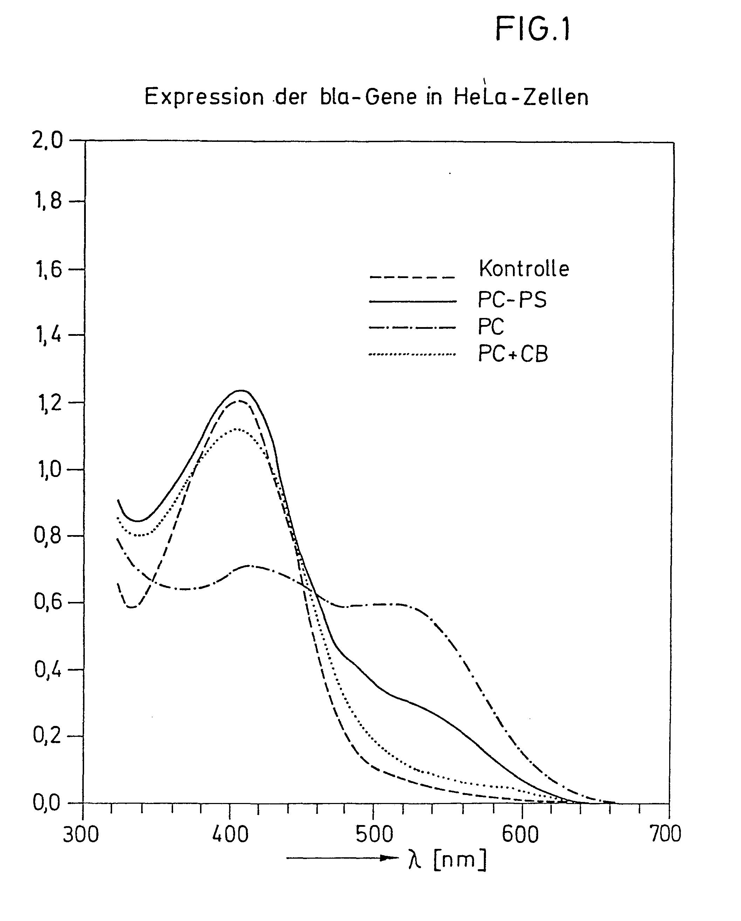

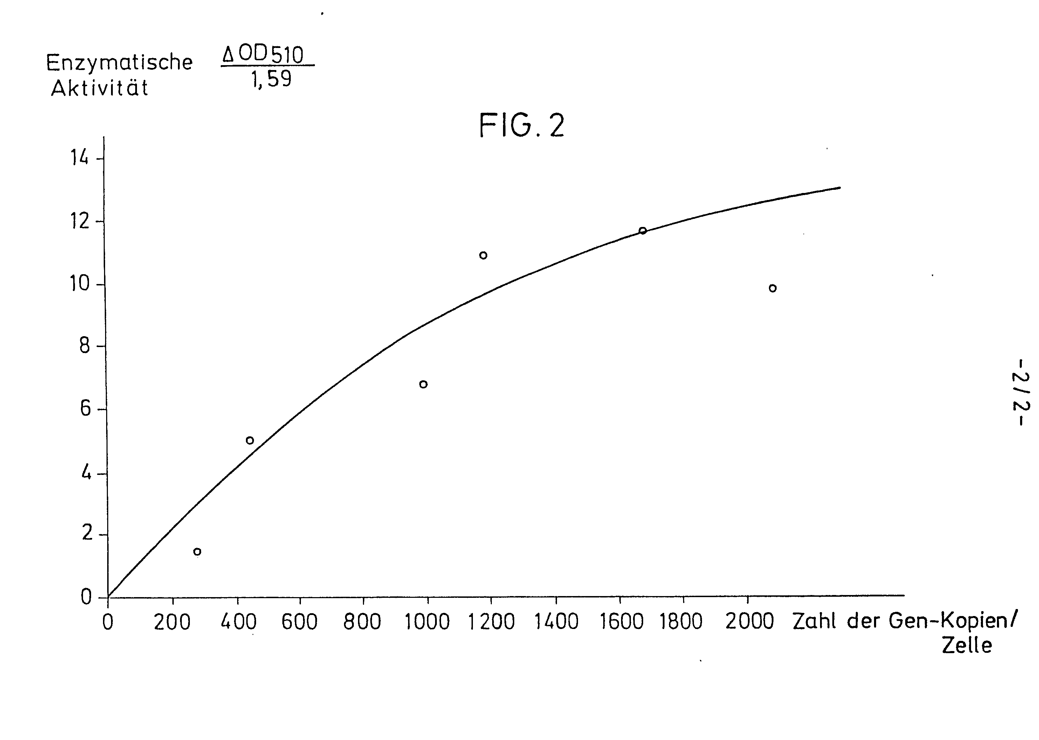

Tabelle I zeigt die Aufnahme der $^{32}$P-DNS in Hela-Zellen im exponentiellen Wachstum. Die Inhibition der Endozytose mit 50 µg/Cytochalasin B führt zu einer starken Abnahme der Zahl der $^{32}$P-DNS-Moleküle, die in den zellulären Kern-DNS aufgenommen werden,wenn die Zellen mit Endozytoseliposomen behandelt worden sind. Wenn elektrisch geladene Liposome benutzt werden, d.h. Liposome, die in der Lage sind, sowohl durch Fusion wie auch durch Endozytose in die Zellen einzudringen, dann ist diese Abnahme wesentlich kleiner. Die Behandlung mit Cytochalasin B inhibiert zu 85 % die DNS-Aufnahme durch Endozytose der DNS-beladenen, neutralen Liposome (PC) aber nur zu 36 % diese Aufnahme, wenn elektrisch geladene Liposome benutzt werden. Nicht nur die Aufnahme der $^{32}$P-DNS durch Endozytose der PC-Liposome ist höher, sondern auch die Expression des Genproduktes ist intensiver, wenn diese Liposome benutzt worden sind (Fig. 1). In dem Kontrollversuch wurden Hela-Zellen verwendet, welche nur leere Liposomen aufgenommen hatten. Die Reaktion mit Cephalosporin führt in diesem Fall zu keiner Absorptionsbande bei 510 nm. Die Hela-Zellen, welche das bla-Gen durch die Endozytose von PC-Liposomen aufgenommen haben, zeigen nach Reaktion mit Cephalosporin eine starke Absorption bei 510 nm (OD = 0.60). Die Zellen, die elektrisch geladene, d.h. Fusionsliposome aufgenommen haben, exprimieren das Gen auch, aber beträchtlich schwächer (OD = 0.30). Wenn Endozytose-Liposome benutzt werden, aber die Endozytose durch Behandlung der Zellen mit Cytochalasin B gehemmt wurde, ist die Expression des Gens sehr gering (OD = 0.12). Eine Gen-Dosierung erscheint also möglich: Wenn man verschiedene Gen-Konzentrationen in die Zellen einschleust und die Expression des Gens in Form von Proteinkonzentration oder enzymatischer Aktivität bestimmt, erhält man die Kurve von Fig. 2.

Eine recht starke Zunahme des Genproduktes wird beobachtet, wenn 200 bis etwa 1200 Genkopien pro Zelle eingebaut werden. Über 1200 Genkopien pro Zelle bewirken praktisch keine Erhöhung der Proteinproduktion mehr.

Die Proteinproduktion wurde über 40 Zellgenerationen verfolgt. Sie zeigt keine signifikanten Schwankungen, ist also konstant, und das Zellwachstum ist unbeeinträchtigt. Dadurch erhält man die Möglichkeit, bestimmte Proteine von eukaryoten Zellen in vorauskalkulierbaren Mengen zu produzieren.

## Tabelle I

Durch Liposome hervorgerufene Aufnahme von $^{32}$p-DNA durch HELA-Zellen im expotentialen Wachstum

| Liposome | $^{32}$p-DNA Gesamtaufnahme | $^{32}$p-DNA Aufnahme im Kernextrakt (CPM) | Kernaufnahme als % der gesamten Aufnahme |
|---|---|---|---|
| **A. Endozytose** | | | |
| P.C. | 1064 | 418 | 39 |
| PC-Cytochalasin B | | | |
| 5o µg/ml | 192 | 58 | |
| **B. Endozytose + Fusion** | | | |
| PC - PS (9 : 1) | 860 | 24o | |
| PC - PS + Cytochalasin B 5o µg/ml | 55o | 14o | 25 |

Mechanismus des Eintritts der PC-PS Liposome: ∼36 % Endozytose

∼64 % Fusion

Effizienz der Kernaufnahme A) Endozytose ∼ 39 %

B) Fusion ∼ 25 %

0043075

- 10 -

Tabelle II

Menge von ß-Lactamase, produziert von $10^7$ HeLa-Zellen als Funktion der Liposomeneinführungsmethode

----------------------------------------------------------------

| Liposome | Typ der Aufnahme | Behandlung | ß-Lactamase ng/ml Zellextrakt |
|---|---|---|---|
| PC | Endozytose | keine | 200 |
| PC | Endozytose- gehindert | Cytochalasin B | 33 |
| PC-PS | 36 % Endozytose 64 % Fusion | keine | 100 |

Tabelle II zeigt die relative Produktivität der Hela-Zellen für ß-Lactamase, wenn das Gen über verschiedene Liposomenaufnahmemechanismen eingeschleust wurde.

Synchronisierung der Zellen

Die Zellen wurden in der Mitose (M) und in der $G_1$-Phase des Zellzyklus synchronisiert. Die verschiedenen Einbauniveaus in den Zellkernen sind in der Tabelle III gezeigt. Obwohl die zelluläre Aufnahme von Endozytose-Liposomen in den mitotischen Zellen abnimmt, werden 72 - 75 % der eingebauten DNS-Moleküle in der chromosomalen DNS gefunden. Wenn die Liposome überwiegend durch Fusion aufgenommen werden, findet man etwa 40 % der inkorporierten DNS-Moleküle in der chromosomalen DNS der Zelle. Exponentiell wachsende Zellen und $G_1$-synchronisierte Zellen unterscheiden sich in ihrer Kernaufnahmefähigkeit nur geringfügig. Überraschend ist, daß die DNS-Aufnahme durch die Fusion mit Liposomen recht hoch in der Mitose ist, in der chromosomalen DNS findet man aber nur

- 11 -

46 % dieser DNS. Mit Endozytose-Liposomen findet man dagegen
72 % der eingebauten DNS in der chromosomalen DNS der Zelle.
Dieses zeigt, daß die Lipid-Doppelschicht der Endozytose-
Liposomen die DNS auf ihrem Weg zum Kern mindestens eine
Zeitlang schützt, während mit Fusion-Liposomen die aufgenommene DNS von Anfang an der Wirkung der zytoplasmischen
DNasen ausgesetzt ist. Diese Ergebnisse wurden mit einer
Reihe von Genen erhalten und haben sich als allgemein gültig
mit sehr unterschiedlichen eukaryoten Zellen erwiesen.

Tabelle III

| Art der Liposome | Zelltyp | Einbau i.d.Zellen | Einbau i.d.Kern | % der Kernfraktion |
|---|---|---|---|---|
| PC | Mitose | 3514 | 2547 | 72 |
| | $G_1$ | 4868 | 1829 | 37 |
| | exponentiell wachsende Zellen | 3532 | 1736 | 49 |
| PC/PS | Mitose | 6454 | 2977 | 46 |
| | $G_1$ | 4100 | 166o | 4o |
| | exponentiell wachsende Zellen | 356o | 1862 | 52 |

- 13 -

Beispiel 2

Die c-DNA des Lysozyms

Die c-DNA des Lysozyms wurde in dem Plasmid pBR 322 an die PST I Schnittstelle eingebaut. Das Gen wurde in Injektions-liposome eingeschlossen.

Einschluß des Gens in Liposome

10 µM Eiphosphatidylcholin (Endozytoseliposome) oder eines Gemisches PC : PS (Phosphatidylserin) im molaren Verhältnis 9 : 1 wurden in Äther gelöst. Sie wurden mit Hilfe einer Hamiltonspritze in einer auf 60°C erwärmten Lösung des Gens (2µg) in His-Tris-Puffer injiziert. Der Äther verdampft und die Lipide bilden große Vesikeln, welche die gelöste DNS einschließen. Die Endozytose- und die Fusionsliposome wurden mit Hela-Zellen inkubiert. Die zelluläre DNS wurde wie vorher isoliert und das radioaktive Gen gezählt.

Die Ergebnisse sind in der Tabelle IV enthalten. Die Expression gemessen als enzymatische Aktivität des Lysozyms erfolgte auch wie im Beispiel 1, zusätzlich mit Hilfe der Nothern-Methode (J.C. Alwine, D.J. Kemp, G.R. Stark, Proc. Natl. Acid. Sci. US 74, 5350, 1977) die Zahl der synthetisierten Lysozym-m-RNS Moleküle in den Zellen bestimmt, die das Lysozym-Gen inkorporiert hatten.

- 14 -

## Tabelle IV

Einbau der Lysozym c.DN$^S$ in Hela-Zellen

| Art der Liposomen | Zelltyp | Einbau in Zellen | Einbau in die Kern DNS | % in der Kernfrak- tion |
|---|---|---|---|---|
| PC | Mitose | 2808 | 2150 | 76 |
| (Endozy- tose) | Expr. | 3640 | 1728 | 41 |
| PC-PC | Mitose | 5410 | 2480 | 46 |
| (Fusion) | Expr. | 4080 | 1936 | 40 |

Bei Verwendung von Endozytose-Liposomen wurden 300 Moleküle Lysozym-m-RNS/Zelle gefunden; bei Verwendung von Fusionliposomen wurden in den Hela-Zellen je 180 Moleküle m RNS (Lysozym) pro Zelle produziert.

Die Effizienz des Einbaus mit Endozytose-Liposomen erweist sich noch einmal. Wiederum sind die Endozytose-Liposome am wirksamsten, wenn die Zellen in der Mitose arretiert (synchronisiert) worden sind.

Beispiel 3

Das Kaninchen ß-Globin Gen in pRB 322 Plasmid

Dieses Gen wurde, wie in Beispiel 2, in Injektions-Liposome eingebaut. Die Liposome aus Eiphosphatidylcholin (PC, Endozytose) oder aus PC-PS (9 : 1, Fusion). Die Hela-Zellen

- 15 -

wurden synchronisiert, wie oben beschrieben. Die Einbauergebnisse sind in der Tabelle V dargestellt. Das Gen wurde
mit $^3$H markiert und die Radioaktivität in den Zellen nach
Inkubation mit den verschiedenen Typen von Liposomen gemessen. Nach Isolierung der Kern-DNS wurde in dieser Fraktion
auch die Radioaktivität gemessen.


Tabelle V


Einbau des ß-Globin-Gens in Hela-Zellen


| Art der Liposome | Zelltyp | Einbau in Zellen | Einbau in der Kern-fraktion | % |
|---|---|---|---|---|
| PC | Mitose | 2654 | 2055 | 77 |
| (Endozy-tose) | Exp. | 3452 | 1681 | 49 |
| | | | | |
| PC-PS | Mitose | 5380 | 2445 | 46 |
| (Fusion) | Exp. | 4050 | 1950 | 47 |

Man kann sehen, daß auch in diesem Fall der höchste Einbau
in der Kernfraktion durch Verwendung der Endozytose-
Liposome erreicht wird. Wenn die Zellen aber in der Mitose
synchronisiert (arretiert) werden, kann die Wirksamkeit des Einbaus mit Endozytose-Liposomen wesentlich noch erhöht werden. Die Expression des Gens, als Zahl der Globin m-RNS Moleküle/ Zelle gemessen, zeigt das gleiche Bild wie im Beispiel 2.

Beispiele 4 - 10

Unter Verwendung der auf Seiten 1 und 2 unter 4.) bis 10.) genannten Gene und Durchführung der Reaktionen in der in Beispielen 1 - 3 beschriebenen Weise werden ähnliche Ergebnisse erzielt.

Diese Erfindung zeigt, daß es überraschenderweise möglich ist, mit Hilfe von Endozytose-Liposomen in Mitose-Zellen wesentlich höhere Kopienzahl von Genen einzubauen und zur Expression zu bringen. Die Bestimmung der Zahl der m-RNS-Moleküle, die dadurch gebildet werden, sowie der Protein-Synthese, die anschließend erfolgt, zeigen, daß dieses Verfahren zur Produktion von größeren Proteinmengen geeignet ist. Es muß hinzugefügt werden, daß die synthetisierten Proteine identisch sind mit den natürlichen Produkten dieser Gene, was für die potentielle biologische Wirkung dieser Proteine wichtig ist.

Durch die Erfindung sind die folgenden Anwendungen und gewerblichen Verwertbarkeiten gegeben:

1.) Eine einfache, leicht stadardisierbare Methode zum Einbau von pro- und eukaryotischen Genen jedwelcher Dimension in eukaryote Zellen, z.B. Einschluß von ganzen Plasmid DNS (MG $> 20 \times 10^6$ c.) in Fusions- u. Endozytose-Liposomen.

2.) Die Herbeiführung einer dauerhaften genetischen Veränderung in Eukaryoten-Zellen, die zur ständigen Produktion des gewünschten Proteins führen.

3.) Eine verbesserte Aufnahme des Genmaterials in die Kernfraktionen der Zellen durch Einschleusung dieses Materials in die Zellen mit Hilfe von Endozytose-Liposomen.

4.) Eine verbesserte Aufnahme des Genmaterials durch Endozytose von Gen-beladenen Liposomen durch Mitose-Zellen.

5.) Eine Dosierung des Genproduktes in den eukaryoten Zellen durch Einbau zunehmender Genkopien mit Hilfe von Endozytose-Liposome (oder Fusion-Liposome) in synchronisierten aber auch exponentiell wachsenden Zellen.

6.) Kombination von synchronisierten Zellen in der Mitose mit DNS beladenen Endozytose-Liposomen zwecks Erreichung der höchsten Proteinproduktion durch die so transformierten Zellen.

7.) Verwendung von Endozytose- (oder auch Fusions-) Liposome, mit DNS beladene, zwecks Einschleusung von Genen in Zellen und Organe lebender Organismen in vivo. Die beispielsweise infrage kommenden Gene sind: ß-Globulin, ⍺-Globulin, Insulin, Leberhydrogenase, das Gen für die Synthese der Phenylhydroxylase, Interferon, Gene für Immunoglobulin, etc.

- 18 -

Patentansprüche

1. Verfahren zur Erhöhung der Aufnahme und der Expression von genetischem Material in die Kerne von intakten Zellen, dadurch gekennzeichnet, daß man mit genetischem Material beladene Endozytose-Liposome durch Endozytose in die Zellkerne einführt.

2. Verfahren nach Anspruch 1, dadurch gekennzeichnet, daß man zusätzlich mit genetischem Material beladene Fusions-Liposome durch Fusion in die Zellkerne einführt.

3. Verfahren nach Ansprüchen 1 und 2, dadurch gekennzeichnet, daß man als genetisches Material DNS-Moleküle verwendet.

4. Verfahren nach Ansprüchen 1 und 2, dadurch gekennzeichnet, daß man als genetisches Material bla-Gen, Restriktionsfragmente des bla-Gens aus dem Plasmid pBR 322 E. coli, das gesamte pBR 322 E.coli Plasmid, das Kaninchen ß-Globin-Gen in pBR 322 Plasmid, die Lysozym C-DNA im gesamten pBR 322 Plasmid, das Conalbumin-Gen im pBR 322 Plasmid, das Thymidinkinase-Gen im pBR 322 Plasmid, das SV 40-Virus, das genomiale Interferongen in pBR 322 Plasmid, die c-DNA des Interferons im Plasmid und das Insulin-Gen im Plasmid pBR 322 verwendet.

5. Verfahren nach Ansprüchen 1 - 4, dadurch gekennzeichnet, daß man die mit genetischem Material beladenen Endozytose-Liposome in synchronosierte mitotische Zellen einführt.

# FIG.1

Expression der bla-Gene in HeLa-Zellen

FIG. 2

Enzymatische Aktivität $\dfrac{\Delta OD\,510}{1,59}$

Zahl der Gen-Kopien/ Zelle

-2/2-

0043075